# EUROPEAN PATENT SPECIFICATION

(11) **EP 4 088 686 B1**
(45) Date of publication and mention of the grant of the patent: **07.10.2026**
(21) Application number: 22156658.1
(22) Date of filing: 09.08.2019
(51) Int. Cl.: A61F 2/07

(54) **STENT GRAFT AND METHOD OF FORMING AN ENDOLUMINAL MEDICAL DEVICE**
STENTTRANSPLANTAT UND VERFAHREN ZUR FORMUNG EINER ENDOLUMINALEN MEDIZINISCHEN VORRICHTUNG
GREFFE D'ENDOPROTHÈSE ET PROCÉDÉ DE FORMATION D'UN DISPOSITIF MÉDICAL ENDOLUMINAL

(30) Priority: 09.08.2018 US 201862716444 P; 09.08.2018 AU 2018214103
(43) Date of publication of application: 16.11.2022
(62) Document number(s) of the earlier application(s) in accordance with Art. 76 EPC: 19191099.1 / 3 646 817
(73) Proprietor: Cook Medical Technologies LLC, Bloomington, IN 47404 (US)
(72) Inventor: KING, Chantelle, Woodridge, 4114 (AU); BRADWAY, Ryan, Lafayette, 47901 (US); KRATZBERG, Jarin, West Lafayette, 47906 (US); WILGER, Kevin, Lafayette, 47905 (US)
(74) Representative: Williams Powell

(56) References cited:
- EP-A1- 3 069 696
- US-A1- 2009 149 939
- US-A1- 2013 306 232
- US-A1- 2016 235 517
- US-B1- 6 814 748

## Description

### TECHNICAL FIELD

The present invention relates generally to stent-grafts and their manufacture.

### BACKGROUND ART

Stents grafts for insertion into a body passageway, such as a vascular passageway or other body lumen are used in situations where the passageway or lumen may have a defect, such as a stenosis or an aneurysm. Stents perform functions that make them useful in combination with grafts. A stent can resist pressure on a graft, and help to provide support for a blood vessel. A stent can provide an outward pressure to keep the graft and hence the lumen open. A stent can also be self-expanding so as to deploy a stent-graft from a compressed and radially compact condition to an expanded deployed condition. A stent may also be used to anchor the graft in place, where anchors or barbs on the stent are provided. Such anchors can secure the stent, and hence the graft, in the desired location within a lumen of a patient.

Typically, stents are attached to grafts using sutures.

A difficulty with known stent-grafts is the complexity and assembly process time required for manufacture. Complexity introduces potential for mistakes.

A further issue with some known stent-grafts is bio-burden. That is, some stent-grafts are known to occasionally release suture material into a patient.

A yet further issue with some known stent-grafts is residual holes left in the graft after temporary or tacking stitches are removed.

US2013306232 A1 relates to devices and methods for attaching support frames to substrates to form articles such as implantable stent-grafts.

### SUMMARY OF THE PRESENT INVENTION

The present invention is as set forth in the appended claims and seeks to provide an improved endoluminal prosthetic device or other medical device, such as a stent graft, and an improved method of forming an endoluminal prosthetic device.

According to an aspect of the invention, there is provided a stent-graft comprising:
a graft having an elongate lumen and a longitudinal axis;
at least one stent having a plurality of struts and an apex between each of the plurality struts, the apices including proximal apices and distal apices;
a running suture attaching the stent to the graft, the running suture comprising:
   first and second knots knotted along the length of each of at least some struts of the plurality of struts at intermediate points spaced from the proximal and distal apices of said at least some struts,
   a length of running suture running between the first and second knots, and a first bridging portion extending between a first knot on a first strut and a first knot on a second strut next adjacent the first strut, and a second bridging portion extending from a second knot on the second strut to a second knot of a third strut next adjacent the second strut.

Advantageously, the first bridging portion is proximal to the second bridging portion.

Preferably, the first bridging portion extends between two struts of a proximal apex and the second bridging portion extends between two struts of a distal apex.

The first bridging portion preferably extends between a first strut of a first proximal apex and a first strut of a second proximal apex.

A length of the bridging portions may pass in and out of the graft between next adjacent struts.

The stent graft may comprise a series of proximal tack knots securing the one or more of the proximal apices of the stent to the graft; and/or a series of distal tack knots securing one or more of the distal apices of the stent to the graft.

Preferably, the series of proximal and/or distal tack knots are configured such that the stent can slide relative to the graft.

The stent graft may comprise a third knot in the running suture between each of the first and second knots.

In the preferred embodiments, the bridging portions are disposed transverse to the longitudinal axis of the lumen.

Each strut of the plurality of struts advantageously has a first knot and a second knot and a first bridging portion extending from the first knot and a second bridging portion extending from the second knot.

It is preferred that the running suture is a single continuous length of suture.

According to another aspect of the present invention, there is provided a stent-graft comprising:
a graft having an elongate lumen and a longitudinal axis;
a plurality of discrete ring stents having a plurality of struts and apices between the struts, the apices including proximal apices and distal apices;
a running suture attaching at least one stent of the plurality of stents to the graft, the running suture comprising:
   at least first and second knots knotted along the length of each strut at intermediate points on the struts away from the proximal and distal apices, a length of running suture running between the first and a second knots on each strut, and
   a first bridging portion extending between a first knot on a first strut adjacent a proximal apex and a first knot on a second strut adjacent the proximal apex, and a second bridging portion extending from a second knot on a second strut adjacent a distal apex to a second knot on a third strut adjacent the distal apex.

Advantageously, the first bridging portion and the second bridging portion are disposed transverse to the longitudinal axis of the lumen; and/or
at least a portion of the first and second bridging portions passes through the graft; and/or
the running suture is continuous about a circumference of the at least one stent; and/or
each stent of the plurality of stents comprises a running suture having first and second knots and first and second bridging portions.

According to another aspect of the present invention, there is provided an aortic stent-graft comprising:
a graft having an elongate lumen and a longitudinal axis;
an external zigzag stent having a plurality of struts and apices between the struts, the apices including proximal apices and distal apices, the stent extending around an external circumference of the graft;
a set of removable proximal sutured knots, the proximal sutured knots securing the proximal apices of the stent to the graft;
a set of removable distal sutured knots, the distal sutured knots securing the distal apices of the stent to the graft; and
a plurality of intermediate sutured knots separate from the set of removable proximal and distal knots, formed along a running suture, the running suture including a plurality of bridging portions, the bridging portions disposed transverse to the longitudinal axis of the lumen and bridging between adjacent struts of the stent, the intermediate sutured knots securing struts of the stent to the graft.

Advantageously, the plurality of bridging portions passes into and out of the graft between adjacent struts of the external zig zag stent and/or the running suture is continuous about a circumference of the external zig zag stent.

According to an aspect of the invention, there is provided a stent-graft comprising:
a graft having an elongate lumen and a longitudinal axis;
an external stent having a plurality of struts and apices between the struts, the apices including proximal apices and distal apices;
a set of proximal sutured knots, the proximal sutured knots securing the proximal apices of the stent to the graft;
a set of distal sutured knots, the distal sutured knots securing the distal apices of the stent to the graft; and
a plurality of intermediate sutured knots, formed along a continuous suture, the continuous suture including a plurality of bridging portions, the bridging portions bridging between neighbouring struts of the stent, the intermediate sutured knots securing struts of the stent to the graft.

All aspects of the present invention, may have one or more of the following characteristics.

Advantageously, the bridging portions are disposed transverse to the longitudinal axis of the lumen.

The plurality of intermediate sutured knots along the continuous suture may include two or three intermediate sutured knots along each strut of the external stent.

The stent is may be zigzag stent. The stent is advantageously self-expanding.

The zigzag stent preferably extends around an external circumference of the graft.

Advantageously, the proximal sutured knots and the distal sutured knots are locking knots.

The intermediate sutured knots may be over-threaded knots.

Preferably, the stent-graft is an aortic stent-graft.

The bridging portions may pass into and out of the graft such that they include external bridging portions and internal bridging portions.

Preferably, the stent-graft comprises a plurality of external stents disposed longitudinally along the graft, each stent having a plurality of struts and apices between the struts, the apices including proximal apices and distal apices thereby forming peaks and valleys, wherein adjacent stents are positioned such that peaks from one of the plurality of external stents extend towards or into valleys of another of the plurality of external stents.

Advantageously, the adjacent stents are positioned such that peaks from one of the plurality of external stents extend into valleys of another of the plurality of external stents.

According to another aspect of the invention, there is provided a method of forming an endoluminal prosthetic device, the method comprising the steps of:
providing a graft comprising a bio-compatible material and defining a main tubular body;
providing a first external stent comprising a plurality of struts and apices between the struts, the apices including proximal apices and distal apices;
positioning the first stent around the main tubular body;
joining the proximal apices and the distal apices of the first stent to the graft using respective sutured proximal knots and sutured distal knots;
joining the struts of the first stent to the graft by intermediate sutured knots in a continuous suture, the continuous suture bridging between neighbouring struts of the stent.

The step of joining the proximal and distal apices of the first stent to the graft may comprise joining using locking knots.

Advantageously, the step of joining the struts of the first stent to the graft by knots in a continuous suture comprises passing the continuous suture into and out of the graft so as to bridge between neighbouring struts of the stent.

The preferred embodiments will be particularly discussed in relation to stent-grafts for placement into the thoracic abdominal aorta or into the abdominal aorta for the treatment of aneurysms. The teachings herein are not, however, so restricted and may be applied to stent-grafts or any other medical device for placement in any lumen of the human or animal body.
the teachings herein can provide an improved stent graft, other medical device, and an improved method of forming an endoluminal medical device, while improving safety and simplifying assembly.

Other aspects and advantages of the teachings herein will become apparent to the skilled person having regard to the specific description that follows.

### BRIEF DESCRIPTION OF THE DRAWINGS

Embodiments of the present invention are described below, by way of example only, with reference to the accompanying drawings, in which:
Figure 1 is a diagrammatic isometric view of a stent-graft according to an embodiment of the invention;
Figure 2 is a detailed view of a portion of the stent-graft shown in Figure 1;
Figures 3A and 3B are a similar view to that of Figure 2, showing a detailed view of a stent-graft according to another embodiment of the invention;
Figure 4 is a similar view to that of Figure 2, but shows a detailed view of a stent-graft according to another embodiment of the invention;
Figure 5 is a detailed view showing a proximal apex of a stent and a proximal locking knot on a stent-graft according to embodiments of the invention;
Figure 6A is an annotated schematic diagram of the suture forming the locking knot of Figure 5;
Figures 6B and 6C are detailed views of a strut of a stent on a stent-graft according to embodiments of the invention;
Figure 6D is a view showing the tightened suture of the embodiment of Figures 6A to 6C;
Figure 7A is a detailed view of a strut of a stent on a stent-graft according to another embodiment of the invention;
Figure 7B is a view showing the tightened suture of the embodiment of Figure 7A;
Figure 8 is a schematic view similar to that of Figure 5, showing another embodiment of locking knot of the tightened suture of the embodiment of Figure 7A;
Figures 9A to 9C are schematic diagrams showing a method of forming the suture arrangement of Figures 7 and 8;
Figure 10 shows a stent-graft according to the invention being deployed from a delivery device;
Figures 11A and 11B an example of branched stent graft in a curved in-use configuration and a straight configuration, respectively;
Figure 12 is a schematic diagram of a prior art suture stent fixation arrangement;
Figures 13A to 13C show a stent graft having a sutured stent according to Figure 12 will curve in use;
Figure 14 is a schematic diagram of a suture stent fixation arrangement as taught herein;
Figures 14A to 14C show a stent graft having a sutured stent according to Figure 14 will curve in use;
Figure 15 is a schematic diagram of a stent graft with stents attached to the graft according to the teachings herein; and
Figure 16 is a schematic diagram of another embodiment of stent graft with stents attached to the graft according to the teachings herein.

### DESCRIPTION OF PREFERRED EMBODIMENTS

For the purposes of understanding the principles of the teachings herein, reference will now be made to the embodiments illustrated in the drawings, and specific language will be used to describe them. It is to be understood that the Figures are, in some cases, schematic and do not show the various components in their actual scale. In many instances, the Figures show scaled up components to assist the reader.

Throughout this specification, the term "distal" with respect to a portion of the aorta, a deployment device or an endograft means the end of the aorta, deployment device or endograft further away in the direction of blood flow from the heart and the term "proximal" means the portion of the aorta deployment device or end of the endograft nearer to the heart in the direction of blood flow. When applied to other vessels, similar terms such as caudal and cranial should be understood.

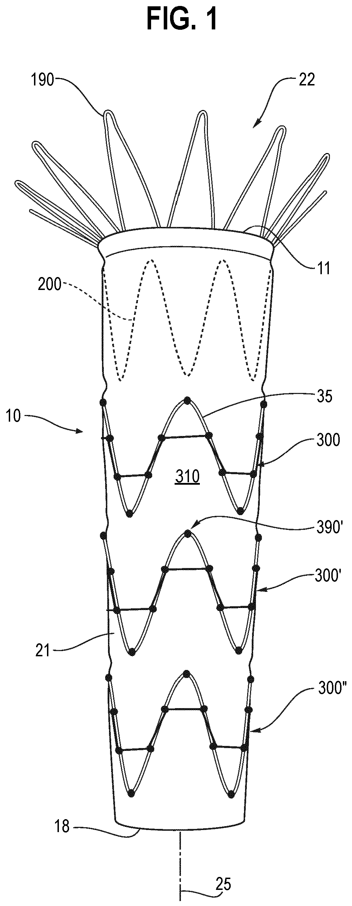

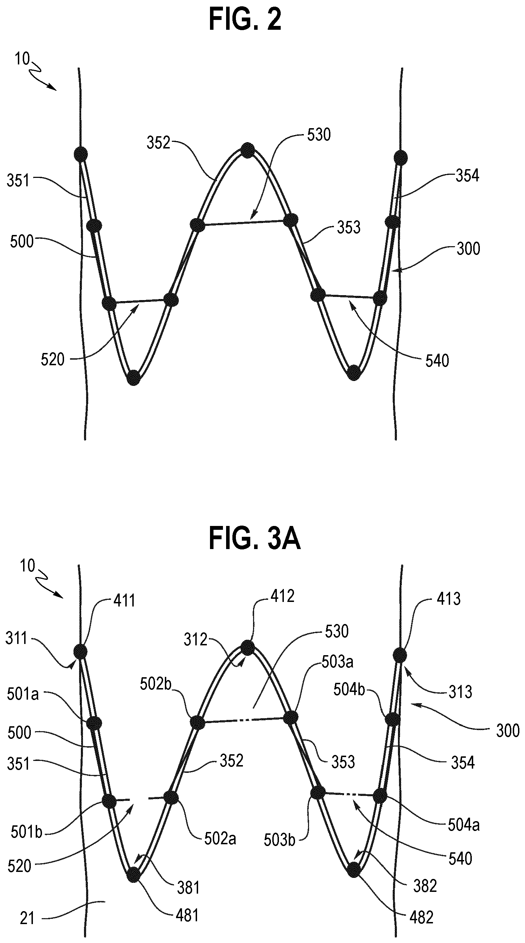

Referring to Figure 1, a stent-graft 10 having a proximal end 11 and a distal end 18 is shown. The stent-graft 10 comprises a graft 20 providing an elongate lumen 22 having a longitudinal axis 25 and one or more external stents 300. Now turning to Figure 2, it can be seen that the external stent 300 has a plurality of struts 351, 352, 353, 354 and apices between the struts, the apices including proximal apices 311, 312, 313 and distal apices 381, 382.

In the embodiment of Figure 1 the stent 300 is a zigzag stent and that the zigzag stent is self-expanding. In other embodiments of the invention, not shown, stents having different geometries may be used.

The zigzag stent 300 extends around an external circumference of the graft 20. Furthermore, it can be seen that there are a plurality of external stents disposed longitudinally along the graft, in this case three (3), each stent having a plurality of struts and apices between the struts. Typical examples of such struts and apices are shown more clearly in Figure 2 as has been described above. The apices include proximal apices and distal apices thereby forming peaks and valleys, wherein adjacent stents are positioned such that peaks from one of the plurality of external stents extend towards or into valleys of another of the plurality of external stents. In Figure 1 stent 300 forms a valley 310 into which peak 390' of stent 300' extends towards. In other embodiments adjacent struts are attached such that there is some overlap and where the peaks extend into valleys of and adjacent stent. Depending on requirements including flexibility, kink resistance, and preventing adjacent stents making metal to metal contact, positioning and overlap of adjacent stents can be varied. The term "valley" is intended to mean the V-shaped space between adjacent struts of a particular stent and applies irrespective of orientation (whether inverted or conventional orientation).

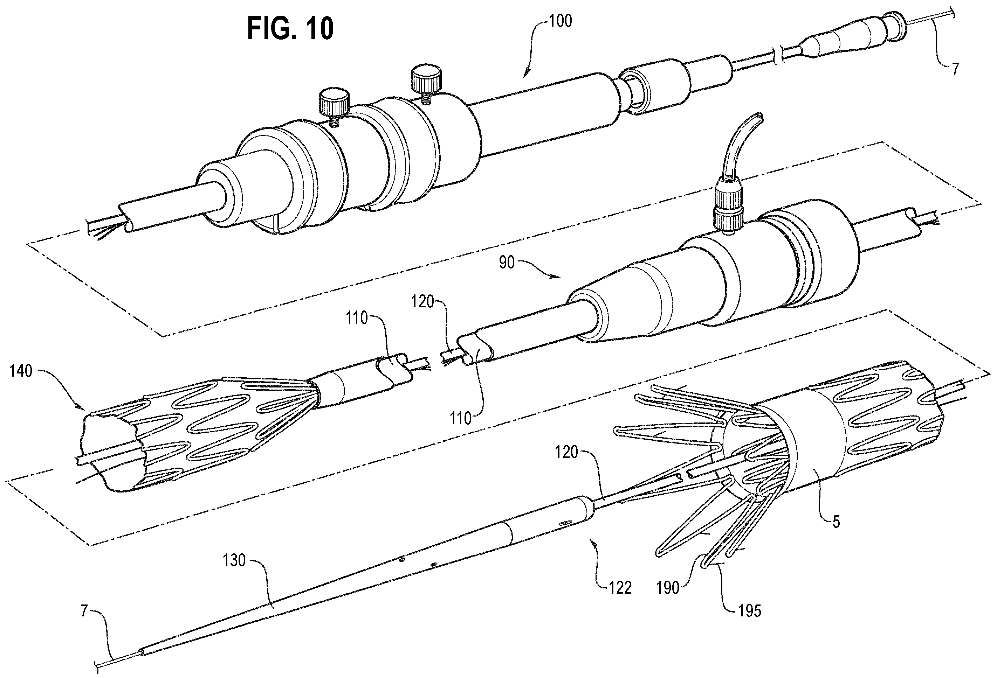

The stent-graft 10 shown in Figure 1 is an aortic stent-graft. However, stent-grafts for other parts of an anatomy may also be provided according to the invention. With the aortic stent-graft 10 of Figure 1, a terminal stent 190 is provided at a proximal end thereof. This terminal stent 190 may be provided with anchoring members in the form of barbs 195, such as those shown in Figure 10. Such barbs 195 help decrease migration of the stent-graft 10 within the vessel in which it is deployed. The stent-graft 10 shown in Figure 1 also includes an internal stent 200 and external stents 300, 300' and 300". Many other stent-graft configurations are possible and may incorporate the features of the teachings disclosed herein.

While the stents 300 are shown external to the graft tubing in these embodiments, it is to be understood that they may be disposed internally of the graft tubing, either all or one or more thereof.

Now referring to Figure 3A, it can be seen that the stent-graft comprises a set of proximal sutured knots 411, 412, 413, the proximal sutured knots securing the proximal apices 311, 312, 313 of the stent 300 to the graft 20. The stent-graft also comprises a set of distal sutured knots 481, 482, the distal sutured knots 481,482 securing the distal apices 381, 382 of the stent 300 to the graft 20. As explained in further detail below, the proximal and distal sutured knots 411-413 and 481-482 may in at least some embodiments be omitted, be removable or be loose sutures, allowing for the stent 300 to slide within the remaining sutures of the assembly.

A plurality of intermediate sutured knots 501a, 501b, 502a, 502b, 503a, 503b, 504a, 504b, formed along a continuous suture 500, are provided. The continuous suture 500 includes a plurality of bridging portions 520, 530, 540 bridging between neighbouring struts of the stent 300. The intermediate sutured knots secure the struts of the stent to the graft 20.

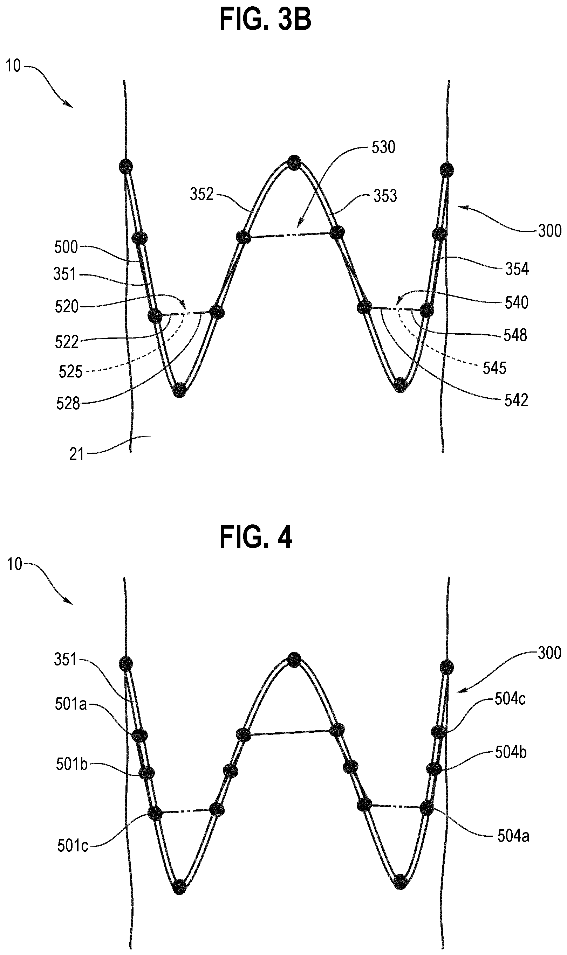

In the embodiment of Figures 3A and 3B, the bridging portions 520, 530, 540 pass into and out of the graft 20 such that they include external bridging portions such as 522 and 528 as shown in Figure 3B, and internal bridging portions, such as 525 also shown in Figure 3B.

With the embodiments shown in Figures 3A and 3B, where the suture has a path through the wall of the graft 20 into and out of the graft 20, so as to bridge between neighbouring struts of the stent, there is less risk of the suture 500 catching.

Looking at bridging portion 520 in Figures 3A and 3B, it can be seen that the bridging portion 520 bridges between knots 501b and 502a substantially circumferentially around a portion of the stent-graft between struts 351 and 352. The bridging portion 520 is disposed at almost exactly 90 degrees to the longitudinal axis 25 of the lumen (as shown in Figure 1). In other embodiments, the angle at which the bridging portions are disposed transverse to the longitudinal axis of the lumen may vary. That is, they may be transverse at an angle other than 90 degrees, but still such that they bridge transversely across to the adjacent strut, rather than following along the strut and around its apex or point.

When Figure 1 is viewed together with Figure 2, it can be seen that the bridging portions 520, 530, 540 are in this embodiment disposed transverse to the longitudinal axis 25 of the lumen 22.

Again referring to Figures 1 and 2, it can be seen that the plurality of intermediate sutured knots along the continuous suture 500 include two intermediate sutured knots along each strut of the external stent 300. In contrast, in another embodiment of the invention shown in Figure 4, there are three intermediate sutured knots along each strut of the external stent. For instance, Figure 4 shows knots 501a, 501b and 501c along strut 351 of the external stent 300. In yet other embodiments (not shown), four or more intermediate sutured knots along each strut of the external stent may be provided depending on factors such as the size of the stent-graft. The intermediate sutured knots are preferably spaced evenly along their corresponding strut at 4 to 7 mm apart.

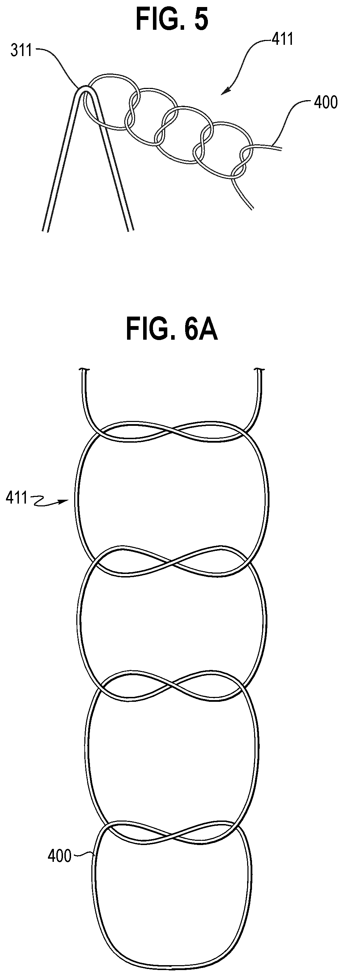

Now referring to Figure 5, a proximal sutured knot 411 is shown in more detail. This proximal sutured knot 411 secures an apex 311 to the graft 20 with a locking knot formed by two pairs of double knots, sometimes referred to as reef knots. The proximal sutured knot 411 is a locking knot, as opposed to a tacking knot. It is a single stitch though the graft material, but has four knots (two pairs of double knots). The knots are a left over right knot, then a right over left knot, then a left over right knot and finally a right over left knot, as can be seen clearly in Figures 5 and 6A. Unlike with some prior art stent-grafts, this knot does not need to be removed.

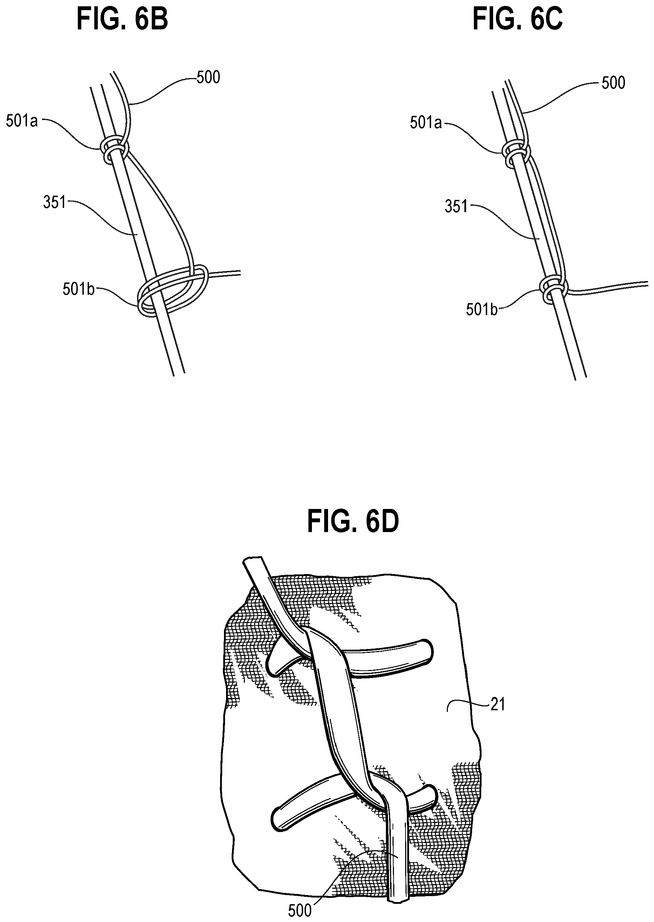

Figures 6B and 6C show a portion of the continuous suture 500 along a strut in a magnified view. In Figure 6B, a first intermediate knot 501a has been tied and a second intermediate knot 501b is in the first stage of being tied. In Figure 6C, the second intermediate knot 501b is still partially tied ready for a second knot so that it will match the first intermediate knot 501a. Figure 6D, shows the continuous suture 500 tied to a graft 21 and in particular the arrangement of the suture at the knots depicted in Figures 6B and 6C.

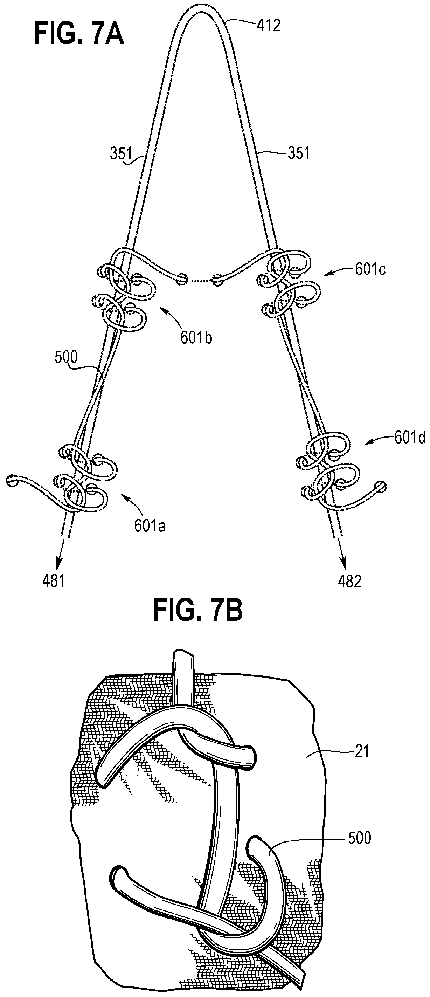

Referring now to Figures 7A and 7B, these show another embodiment of continuous suture with a modified knot arrangement compared to that of the embodiment of Figures 5 to 6D. In the embodiment of Figures 7A and 7B, four double knot arrangements 601a-601d are formed on the stent struts 351 intermediate the apices 412, 481, 482 of the stent. Each double knot of the continuous suture is effectively the same as the other knots 601a-601d of the continuous suture thread 500. The double knot 601a is formed by a loop of suture thread passing out of the graft material 21 and then back into the graft material at the same side of the stent strut 351 and from the other side of graft material passes back out again on the opposite side of the strut 351, to be looped underneath the first formed loop, over the stent strut 351, to enter into the other side of the graft material on the first side of the strut, then looped out again at the other side of the strut and then passed through the formed second loop on the first side of the strut, before passing towards the second double loop arrangement. Figure 7B shows one of the double-loop knots depicted in schematic form in Figure 7A.

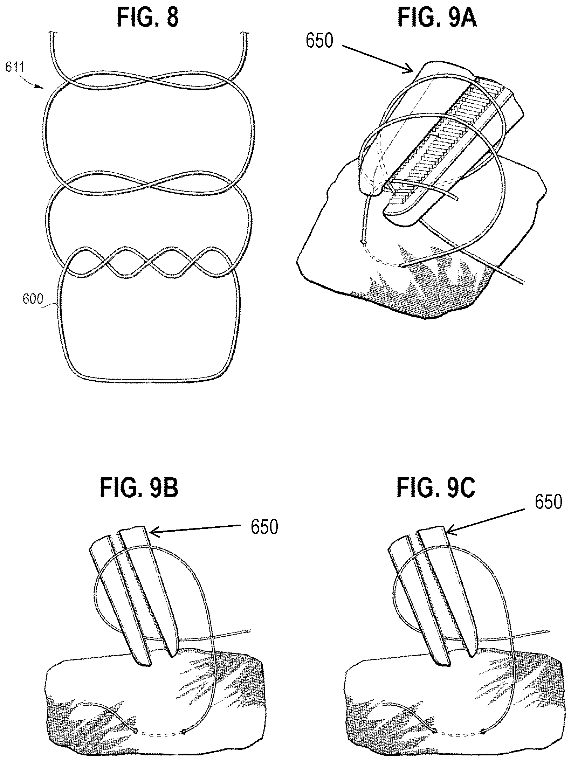

Referring to Figure 8, a proximal sutured knot 611 is shown in more detail. Similar to the embodiment of Figures 5 and 6A, the proximal sutured knot 611 secures an apex 311 to the graft 20 with a locking knot formed by two pairs of double knots, sometimes referred to as reef knots. This provides a staggered stitch with, for each double knot, a double formed left over right stitch, as can be seen in Figure 8. The staggered stitch is fast to sew and reduces the number of times the manufacturer has to swap tools to complete the sewing process. This can help with cross-contamination from handling tools too often and requires less time to be spent handling the graft during manufacture thereof.

Referring now to Figures 9A to 9C, these depict how the double knot arrangement of Figures 7A to 8 can be achieved in one manufacturing implementation. With reference to Figure 9A, the first knot is looped twice around needle holders 650 and tightened. With reference to Figure 9B, the second knot is looped once around the needle holders 650 and tightened, while, with reference to Figure 9C, the third knot is looped once around the needle holders 650 and tightened. Thereafter, the suture tails are cut with a finishing length of, preferably, around 3.0mm plus or minus 1.0mm.

Turning now to Figure 10, a delivery assembly 100 sliding along a guide wire 7 and having an introducer 90 is shown. Such a delivery assembly 100 can be used to deliver a stent-graft according to the teachings herein. The delivery assembly 100 comprises a guide wire catheter 120 having a tip 130 mounted to a proximal end 122 thereof. A distal delivery member 110, which could be referred to as a pusher, is mounted around the guide wire catheter 120 and is located distal of the tip 130. A stent-graft receiving portion 140 is provided between the tip 130 and the distal delivery member 110. The delivery assembly 100 is slidably mounted through the introducer 90. A handle 150 is manipulable to slide the distal delivery member (pusher) 110 through the introducer 90 so as to position the stent-graft 5 in the vascular system of a patient.

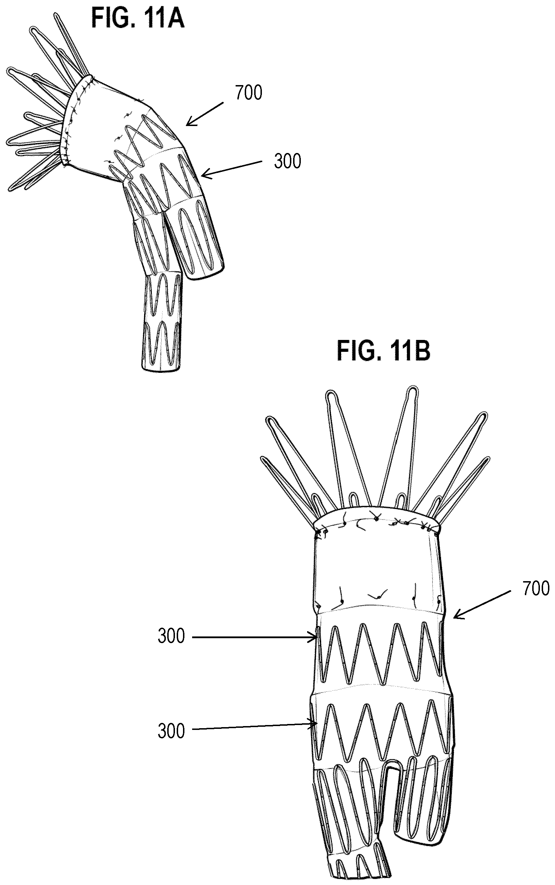

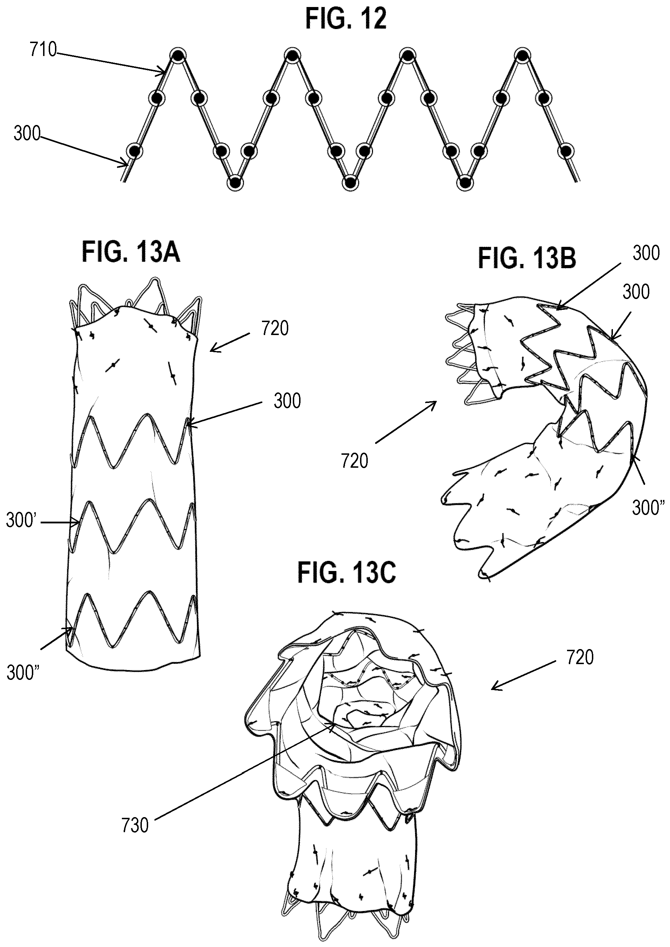

Figures 11A and 11B show a stent graft having a plurality of stents 300 attached to the graft material in a conventional manner, as shown in Figure 12, that is with a running suture 700 extending along each strut of the stent and to each apex. The stent is firmly attached, which results in stiffness of the stent graft 700 to bending, as will be apparent from Figure 11A and as is known in the art.

Referring now to Figures 13A to 13B, these show another embodiment of stent graft 720 that again has the stents attached to the graft material by a running suture that extends along the entirety of each strut to the apices thereof. The stents in this example are spaced form one another by around 10 millimetres. As can be seen in particular in Figures 13B and 13C, while the stent spacing allows greater bending of the stent graft compared to the example of Figures 11A and 11B, when curved at a relatively tight angle, as might occur for example in the aortic arch, significant ruffling 730 of the graft material occurs particularly at the inside of the curve. This ruffling can cause significant turbulence in the flow of fluid (blood) through the stent graft, disrupting normal blood flow and function.

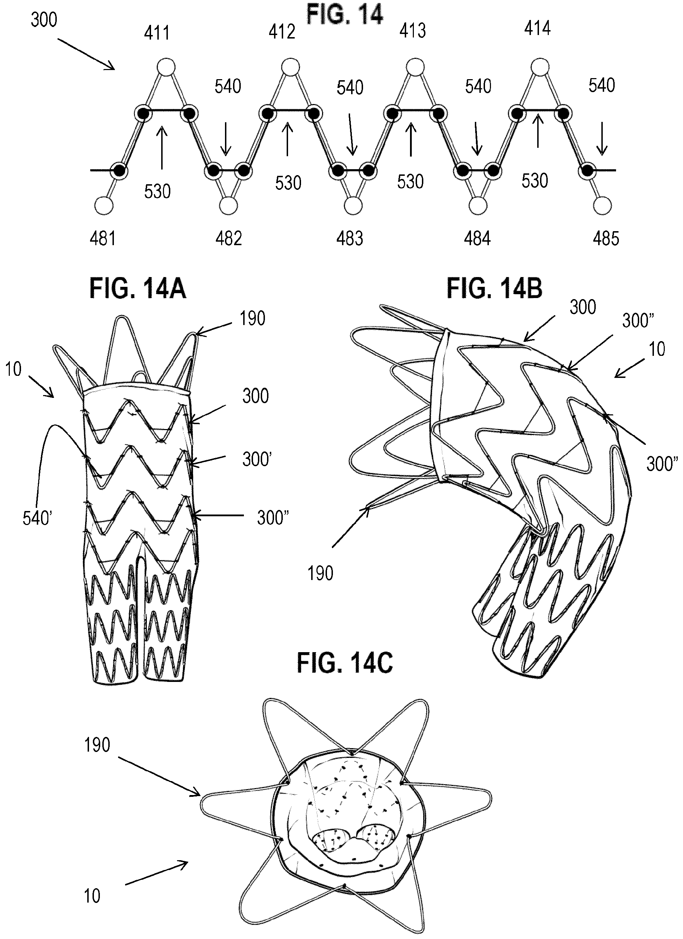

Referring now to Figure 14, this shows in schematic form another embodiment of stent attachment arrangement similar to that of Figure 2 but in which the stent apices 411-414 and 481-485 do not have any sutures attaching them to the graft material. In other words, the stent 300 is attached to the graft tubing solely by the intermediate running suture 500. This may be achieved by use of tack knots that may be removed, degrade or by omitting them entirely. In other embodiments, only loose tack knots may be used that allow sliding of the stent within the constraints of the intermediate running suture.

While the embodiment of Figure 14 shows neither the proximal nor the distal apices 411-414 and 481-485 having any tack knots, in practice it may be preferred to maintain the proximal tack knots and remove the distal tack knots.

Referring now to Figures 14A to 14C, this shows an embodiment similar to that of Figure 14 but in which the proximal bridging portions 530 are disposed inside the graft tubing, which is an alternative to the arrangement shown in figure 14. In practice, the bridging portions 530, 540 may weave into and out of the graft material so as in effect to be held by the material. The intermediate suture arrangement assist in the control of the graft material as the stent graft 10 is curved, as it would be in a curved vessel, and in particular prevents or substantially limits ruffling of graft material at the interior of the curve, as can be seen by a comparison of Figure 14C with Figure 13C.

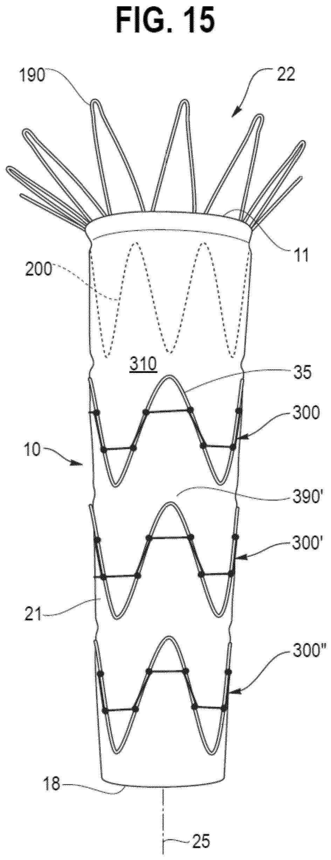

Figure 15 shows a stent graft provided with three stents 300, 300' and 300" having the intermediate suture arrangement of Figure 14 and free, that is non-attached, stent apices.

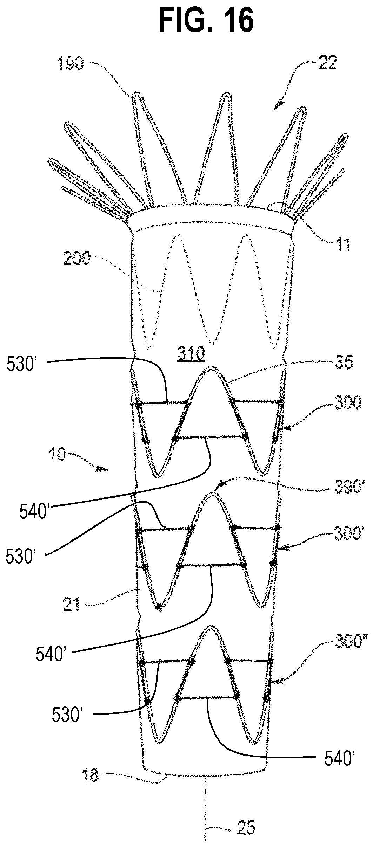

Figure 16 shows a slightly modified embodiment, in which the bridging portions 530' and 540' are arranged between adjacent apices of the stents 300, 300', 300", that is at the wider ends of the V formed by adjacent struts of the stent.

Preferably, the graft 20 is formed from tube material may be formed from a biocompatible material that is substantially non-toxic in the *in vivo* environment of its intended use and will be substantially not rejected by the patient's physiological system (i.e., is non-antigenic). For example, the graft tube material may be made of an expanded polytetrafluoroethylene (ePTFE), polytetrafluoroethylene, silicone, polyurethane, polyamide (nylon), polyethylene, polypropylene, polyaramids, polyacrylonitrile, cellulose, or another flexible biocompatible material. The graft tube material also may be made of known fabric graft materials, e.g., woven polyester such as DACRON.RTM. from Invista (Wichita, Kans.), polyetherurethanes such as THORALON.RTM. from Thoratec Corporation (Pleasanton, Calif.), or polyethylene such as an ultra-high molecular weight polyethylene (UHMwPE) such as DYNEEMA.RTM. from DSM Dyneema LLC (Stanley, N.C.). In addition, materials that are not inherently biocompatible may be subjected to surface modifications to render the materials biocompatible. Examples of surface modifications include, for example, graft polymerization of biocompatible polymers on the surface, coating of the surface with a crosslinked biocompatible polymer, chemical modification with biocompatible functional groups, or immobilization of a compatibilizing agent such as heparin or other biocompatible substances. Thus, any fibrous material having sufficient strength to survive in the *in vivo* environment may be used to form a textile graft, provided the final textile is biocompatible.

The graft tube material may also include a bio-remodelable material such as reconstituted or naturally-derived collagenous materials. Suitable remodelable materials can be provided by collagenous extracellular matrix (ECM) materials possessing biotropic properties. For example, suitable collagenous materials may include ECM materials such as those comprising submucosa, renal capsule membrane, dermal collagen, dura mater, pericardium, fascia lata, serosa, peritoneum or basement membrane layers, including liver basement membrane. Suitable submucosa materials for these purposes may include, for instance, intestinal submucosa including small intestinal submucosa, stomach submucosa, urinary bladder submucosa and uterine submucosa. Collagenous matrices including submucosa (potentially along with other associated tissues) useful in the present invention can be obtained by harvesting such tissue sources and delaminating the submucosa-containing matrix from smooth muscle layers, mucosal layers, and/or other layers occurring in the tissue source. For additional information as to some of the materials useful in the present invention, and their isolation and treatment, reference can be made, for example, to US Patent numbers 4,902,508, 5,554,389, 5,993,844, 6,206,931, and 6,099,567. A non-limiting example of a suitable remodelable material may include SURGISIS^{RTM} BIODESIGN^{™} from Cook Medical (Bloomington, Ind.) or the graft prosthesis material described in US Patent Number 6,206,931 to Cook et al.. The graft tube material also may be made of any of the materials described in US Patent Number 7,407,509 to Greenberg et al. or US Patent Application Publication Number 2009/0171451 to Kuppurathanam et al..

The stents of the embodiments of the invention, such as stents 200, 300, 300' and 300", may have any suitable stent pattern known in the art. The stents may be balloon expandable. Preferably, the stents may be self-expandable. The stents can maintain the patency of the prosthesis and ensure adequate sealing against the surrounding vascular tissue. One goal for stent design and placement, whether internal or external, may be to prevent metal-to-metal contact points, prevent contact between two different types of alloys, and minimize micro-motion. The arrangement shown in Figure 1, where adjacent stents are positioned such that peaks from one of the plurality of external stents extend towards the valleys of another of the plurality of external stents, assist in preventing metal-to-metal contact points. Stent sizing, spacing, and design may be determined so that there is no stent-to-stent contact even in tortuous anatomy. Stents preferably may be placed to maximize prosthesis flexibility while maintaining patency, as well as reduce material wear and stent fatigue. Furthermore, it is preferable that the stents do not interfere with the branch, that they minimize the potential for galvanic corrosion and ensure adequate joint stability. Stent amplitude, spacing, and stagger preferably may be optimized for each prosthesis design. Any of the stents mentioned herein may have barbs and/or other anchoring members to help decrease prosthesis migration.

One example of a stent pattern is the Z-stent or Gianturco stent design. Each Z-stent may include a series of substantially straight segments or struts interconnected by a series of bent segments or bends. The bent segments may include acute bends or apices. The Z-stents are arranged in a zigzag configuration in which the straight segments are set at angles relative to one another and are connected by the bent segments. This design provides both significant radial force as well as longitudinal support. In tortuous anatomy, branches or fenestrations, it may be preferable to use alternative stents or modifications to the Z-stent design to avoid stent-to-stent contact. Alternative stents may include, for example, annular or helical stents. Furthermore, in complex anatomical situations, external stents may have the potential to become intertwined with the wires or other devices utilized to ensure branch vessel access, sealing, and fixation. Thus, in some instances, it may be desirable to affix some of the stents to the internal surface of the prosthesis.

The stents described herein may be made from any suitable material known in the art. In one example, the stents may be made from standard medical grade stainless steel and are soldered using silver standard solder (0 lead/0 tin). In other examples, the stents may be made from a metallic material selected from any type of stainless steel, silver, platinum, palladium, gold, titanium, tantalum, iridium, tungsten, cobalt, chromium, cobalt-chromium alloy 1058, cobalt-based 35N alloy, nickel-based alloy 625, a molybdenum alloy, a molybdenum alloy including about 0.4% to about 0.8% of lanthanum oxide (Li.sub.2O.sub.3), and a nickel-titanium alloy, or other suitable materials known in the art. The stents also may be made from nitinol or other shape-memory metal. Moreover, the stents may be configured in a variety of ways to provide a suitable intraluminal support structure. For example, one or more stents may be made from a woven wire structure, a laser-cut cannula, individual interconnected rings, or another pattern or design.

Stent-grafts according to the teachings herein, such as stent-graft 10, can be made efficiently. They do not require temporary stitches that are used commonly in producing other stent-grafts, particularly those having external stents. This saves considerable time and improves manufacturing efficiency. It is also easier to construct as the sewer does not have to turn the graft upside down continuously as they sew along the strut. Instead, the graft can be held in one position (upright) as the suture runs along the graft. Making the process easier reduces errors and the need for re-work.

Aside from being more efficient, avoiding the need for temporary or tacking stitches avoids additional holes being formed in the graft material. Also avoided is the potential for tacking stitches to be missed and then forming a bio-burden with the patient during or after endovascular surgery.

Stent-grafts according to the teachings herein, such as stent-graft 10, provide a safer prosthetic in that cuts to the graft material are much less likely than stent-grafts that utilise temporary stitches (for instance to initially hold apices in place). Avoiding cuts and holes is important because cuts or holes can cause blood leakage into aneurysmal or other areas outside the lumen of the vessel being repaired.

A method of forming an endoluminal prosthetic device, such as is shown in Figures 1 and 2, will now be described.

A graft comprising a bio-compatible material, such as the bio-compatible material described above, defining a main tubular body is provided. A first external stent is also provided, the first external stent comprising a plurality of struts and apices between the struts, the apices including proximal apices and distal apices. The first stent 300 is positioned around the main tubular body (such as is shown in Figures 1 and 2). The proximal apices and the distal apices of the first stent are joined to the graft using respective sutured proximal knots and sutured distal knots. The struts of the first stent are joined to the graft by intermediate sutured knots in a continuous suture, the continuous suture ridging between neighbouring struts of the stent, as is clearly shown in Figure 2.

In one embodiment of a method according to the invention, the step of joining the proximal and distal apices of the first stent to the graft comprises using locking knots, such as the locking knot shown in Figure 5.

In a further method according to the invention, the step of joining the struts of the first stent to the grafts by knots in a continuous suture comprises passing the continuous suture into and out of the graft, such as is shown in Figure 3A.

It is preferred that the intermediate suture 500 that provides the bridging portions 530, 540 is a continuous suture that extends circumferentially around the entirety of the stent 300, 300', 300". This reduces the number of tie knots, optimises compressibility of the stent graft for deployment and facilitates assembly. However, in some embodiments the running intermediate suture 500 may be provided in a plurality of lengths, that is each would extend only partially around the stent 300, 300', 300". It is also possible to have the intermediate sutures 500 as individual bridging portions 530, 540 with the suture tied and terminating at each end of a bridging portion, although this is a less preferred arrangement.

As explained above, one or more or all of the stents 300,300', 300", that is the stents provided with intermediate bridging sutures, could be disposed on the outside of the graft tubing, as in the preferred embodiments described above and shown in the drawings or one or more could be disposed inside the graft tubing.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the invention is not restricted in its use to the particular application described or to stent grafts per se, being applicable to other types of implantable medical devices having a stent or scaffold attached to a support such as a graft material or other membrane. Neither is the present invention restricted in its preferred embodiment with regard to the particular elements and/or features described or depicted herein. It will be appreciated that the invention is not limited to the embodiment or embodiments disclosed, but is capable of numerous rearrangements, modifications and substitutions without departing from the scope of the invention as set forth and defined by the following claims.

The disclosures in United States patent application number 62/716,444 and in Australian patent application number 2018/214103, from which this application claims priority, in European patent application number 19191099.1, from which this application is divided, and in the abstract accompanying this application, are mentioned herein for information purpose.

## Claims

1. A stent-graft (10) comprising:
a graft (20) having a proximal end (11) and a distal end (18) and defining an elongate lumen (22) having a longitudinal axis (25);
an internal stent (200);
at least one body stent (300) distal of the internal stent (200) having a plurality of struts (351-354) and apices (311-313, 381, 382) between each of the plurality struts (351-354), the apices (311-313, 381, 382) including proximal apices (311-313) and distal apices (381, 382);
a first running suture (500) attaching a body stent (300) of the at least one body stent (300, 300', 300") to the graft (20), the first running suture (500) comprising:
a first knot and a second knot (503a, 503b) knotted along a length of a first strut (353) of the plurality of struts (351-354) at intermediate points along the first strut (353) and spaced from the proximal and distal apices (311-313, 381, 382),
a first length of the first running suture (500) extending from the first knot to the second knot (503a, 503b),
a third knot (504a) knotted on a second strut (354) next adjacent the first strut (353) at an intermediate point along the second strut (354) and spaced from the proximal and distal apices (311-313, 381, 382) and a fourth knot (504b) on the second strut (354) proximal of the third knot (504a) at an intermediate point along the second strut (354) and spaced from the proximal and distal apices (311-313, 381, 382),
a first bridging portion (540) extending transversely between the second knot (503b) and the third knot (504a), and
a second length of the first running suture (500) running between the third and fourth knots (504a, 504b),
wherein the running suture (500) is a single continuous suture from the first knot to the fourth knot (503a, 503b, 504a, 504b), and
wherein the first bridging portion (540) is transverse to the longitudinal axis (25).

2. The stent graft of claim 1, wherein the body stent comprises a plurality of pairs of adjacent struts sharing a distal apex, wherein each pair of adjacent struts sharing a distal apex has first, second, third and fourth knots and a second running suture extending distally from the first knot to the second knot, extending transversely to the third knot to form a bridging portion between the second and third knots, and running proximally to the fourth knot.

3. The stent graft of claim **1,** wherein each bridging portion traverses an external portion of the graft for at least one length and an internal portion of the graft for at least one length between the second and third knots.

4. The stent graft of claim 1, wherein each bridging portion extends between two adjacent struts of a distal apex.

5. The stent graft of claim 1, further comprising a fifth suture knot of the running suture disposed between the first and second suture knots and a sixth suture knot of the running suture knot disposed between the third and fourth suture knots.

6. The stent graft of claim 1, wherein a plurality of proximal apices and/or a plurality of distal apices are devoid of suture knots.

## Patentansprüche

1. Stenttransplantat (10), das Folgendes umfasst:
ein Transplantat (20) mit einem proximalen Ende (11) und einem distalen Ende (18), das ein längliches Lumen (22) mit einer Längsachse (25) definiert;
einen inneren Stent (200);
mindestens einen Körperstent (300) distal von dem inneren Stent (200), der mehrere Streben (351-354) und Spitzen (311-313, 381, 382) zwischen jeder der mehreren Streben (351-354) aufweist, wobei die Spitzen (311-313, 381, 382) proximale Spitzen (311-313) und distale Spitzen (381, 382) beinhalten;
eine erste laufende Naht (500), die einen Körperstent (300) des mindestens einen Körperstents (300, 300', 300") an dem Transplantat (20) befestigt, wobei die erste laufende Naht (500) Folgendes umfasst:
einen ersten Knoten und einen zweiten Knoten (503a, 503b), die entlang einer Länge einer ersten Strebe (353) der mehreren Streben (351-354) an Zwischenpunkten entlang der ersten Strebe (353) geknotet sind und von den proximalen und distalen Spitzen (311-313, 381, 382) beabstandet sind,
eine erste Länge der ersten laufenden Naht (500), die sich von dem ersten Knoten zu dem zweiten Knoten (503a, 503b) erstreckt,
einen dritten Knoten (504a), der an einer zweiten Strebe (354) als nächstes angrenzend an die erste Strebe (353) an einem Zwischenpunkt entlang der zweiten Strebe (354) geknotet ist und von den proximalen und distalen Spitzen (311-313, 381, 382) beabstandet ist, und einen vierten Knoten (504b) an der zweiten Strebe (354) proximal zu dem dritten Knoten (504a) an einem Zwischenpunkt entlang der zweiten Strebe (354) und von den proximalen und distalen Spitzen (311-313, 381, 382) beabstandet,
einen ersten Überbrückungsabschnitt (540), der sich quer zwischen dem zweiten Knoten (503b) und dem dritten Knoten (504a) erstreckt, und
eine zweite Länge der ersten laufenden Naht (500), die zwischen dem dritten und dem vierten Knoten (504a, 504b) verläuft,
wobei die laufende Naht (500) eine einzelne durchgehende Naht vom ersten Knoten zum vierten Knoten (503a, 503b, 504a, 504b) ist, und
wobei der erste Überbrückungsabschnitt (540) quer zu der Längsachse (25) ist.

2. Stenttransplantat nach Anspruch 1, wobei der Körperstent mehrere Paare angrenzender Streben umfasst, die sich eine distale Spitze teilen, wobei jedes Paar angrenzender Streben, die sich eine distale Spitze teilen, einen ersten, einen zweiten, einen dritten und einen vierten Knoten und eine zweite laufende Naht aufweist, die sich distal vom ersten Knoten zum zweiten Knoten erstreckt, sich quer zum dritten Knoten erstreckt, um einen Überbrückungsabschnitt zwischen dem zweiten und dem dritten Knoten zu bilden, und proximal zum vierten Knoten verläuft.

3. Stenttransplantat nach Anspruch 1, wobei jeder Brückenabschnitt einen äußeren Abschnitt des Transplantats für mindestens eine Länge und einen inneren Abschnitt des Transplantats für mindestens eine Länge zwischen dem zweiten und dem dritten Knoten durchquert.

4. Stenttransplantat nach Anspruch 1, wobei sich jeder Brückenabschnitt zwischen zwei angrenzenden Streben einer distalen Spitze erstreckt.

5. Stenttransplantat nach Anspruch 1, ferner umfassend einen fünften Nahtknoten der laufenden Naht, der zwischen dem ersten und dem zweiten Nahtknoten angeordnet ist, und einen sechsten Nahtknoten der laufenden Naht, der zwischen dem dritten und dem vierten Nahtknoten angeordnet ist.

6. Stenttransplantat nach Anspruch 1, wobei mehrere proximale Spitzen und/oder mehrere distale Spitzen frei von Nahtknoten sind.

## Revendications

1. Greffe d'endoprothèse (10) comprenant :
une greffe (20) présentant une extrémité proximale (11) et une extrémité distale (18) et définissant une lumière allongée (22) présentant un axe longitudinal (25) ;
une endoprothèse interne (200) ;
au moins une endoprothèse de corps (300) distale par rapport à l'endoprothèse interne (200) présentant une pluralité d'entretoises (351-354) et de sommets (311-313, 381, 382) entre chaque entretoise (351-354), les sommets (311-313, 381, 382) comprenant des sommets proximaux (311-313) et des sommets distaux (381, 382) ;
une première suture continue (500) fixant une endoprothèse de corps (300) de l'au moins une endoprothèse de corps (300, 300', 300'') à la greffe (20), la première suture continue (500) comprenant :
un premier nœud et un deuxième nœud (503a, 503b) noués sur une longueur d'une première entretoise (353) de la pluralité d'entretoises (351-354) au niveau de points intermédiaires le long de la première entretoise (353) et espacés des sommets proximaux et distaux (311-313, 381, 382),
une première longueur de la première suture continue (500) s'étendant du premier nœud au deuxième nœud (503a, 503b),
un troisième nœud (504a) noué sur une seconde entretoise (354) adjacente à la première entretoise (353) au niveau d'un point intermédiaire le long de la seconde entretoise (354) et espacé des sommets proximaux et distaux (311-313, 381, 382) et un quatrième nœud (504b) sur la seconde entretoise (354) proche du troisième nœud (504a) au niveau d'un point intermédiaire le long de la seconde entretoise (354) et espacé des sommets proximaux et distaux (311-313, 381, 382),
une première partie pont (540) s'étendant transversalement entre le deuxième nœud (503b) et le troisième nœud (504a), et
une seconde longueur de la première suture continue (500) s'étendant entre les troisième et quatrième nœuds (504a, 504b),
la suture continue (500) étant une suture continue unique du premier nœud au quatrième nœud (503a, 503b, 504a, 504b), et
la première partie pont (540) étant transversale à l'axe longitudinal (25).

2. Greffe d'endoprothèse selon la revendication 1, l'endoprothèse de corps comprenant une pluralité de paires d'entretoises adjacentes partageant un sommet distal, chaque paire d'entretoises adjacentes partageant un sommet distal présentant des premier, deuxième, troisième et quatrième nœuds et une seconde suture continue s'étendant distalement du premier nœud au deuxième nœud, s'étendant transversalement au troisième nœud pour former une partie pont entre les deuxième et troisième nœuds, et s'étendant proximalement au quatrième nœud.

3. Greffe d'endoprothèse selon la revendication 1, chaque partie pont traversant une partie externe de la greffe sur au moins une longueur et une partie interne de la greffe sur au moins une longueur entre les deuxième et troisième nœuds.

4. Greffe d'endoprothèse selon la revendication 1, chaque partie pont s'étendant entre deux entretoises adjacentes d'un sommet distal.

5. Greffe d'endoprothèse selon la revendication 1, comprenant en outre un cinquième nœud de suture de la suture continue situé entre les premier et deuxième nœuds de suture et un sixième nœud de suture du nœud de suture continue situé entre les troisième et quatrième nœuds de suture.

6. Greffe d'endoprothèse selon la revendication 1, une pluralité de sommets proximaux et/ou une pluralité de sommets distaux étant dépourvus de nœuds de suture.
